# EUROPEAN PATENT SPECIFICATION

(11) **EP 4 144 344 B1**
(45) Date of publication and mention of the grant of the patent: **26.08.2026**
(21) Application number: 20933320.2
(22) Date of filing: 09.10.2020
(51) Int. Cl.: A61K 8/9789, A61K 8/31, A61K 8/63, A61K 8/92, A61K 8/68, A61K 8/34, A61K 8/67, A61Q 19/00, A61Q 19/02, A61Q 19/08

(54) **BIOMIMETIC AND CAMELLIA EXTRACT-CONTAINING SKIN BARRIER REPAIR COMPOSITION AND APPLICATION THEREOF**
BIOMIMETISCHE UND CAMELLIA-EXTRAKTHALTIGE HAUTBARRIEREREPARATURZUSAMMENSETZUNG UND ANWENDUNG DAVON
COMPOSITION DE RÉPARATION DE BARRIÈRE CUTANÉE BIOMIMÉTIQUE CONTENANT UN EXTRAIT DE CAMÉLIA ET SON APPLICATION

(30) Priority: 29.04.2020 CN 202010357988
(43) Date of publication of application: 08.03.2023
(73) Proprietor: Shanghai Forest Cabin Biological-Tech Co., Ltd., Shanghai 315400 (CN)
(72) Inventor: YI, Zhiying, Shanghai 315400 (CN); LUO, Biaoyu, Shanghai 315400 (CN); SANG, Jianmei, Shanghai 315400 (CN); ZHANG, Xiufang, Shanghai 315400 (CN); XIE, Dan, Shanghai 315400 (CN); XIE, Qingbin, Shanghai 315400 (CN); GAO, Hongqi, Shanghai 315400 (CN)
(74) Representative: Metida
(86) International application number: PCT/CN2020/119879
(87) International publication number: WO 2021/218039

(56) References cited:
- EP-A1- 2 133 066
- CN-A- 106 943 537
- CN-A- 107 440 942
- CN-A- 108 743 474
- CN-A- 109 498 475
- CN-A- 110 013 445
- CN-A- 110 384 652
- CN-A- 110 448 486
- CN-A- 110 859 762
- CN-A- 111 557 874
- US-A1- 2012 093 896

## Description

### Technical Field

The present invention belongs to the field of cosmetics, and relates to a camellia extract-containing skin barrier repair composition and an application thereof, particularly to a biomimetic (highly skin-friendly) and camellia extract-containing skin barrier repair composition and an application thereof.

### Background Art

After thousands of years of evolution, our skin has formed an organic whole of dermis, epidermis, and stratum corneum that are efficient and organized, and function day after day and year after year. When the skin is exposed to external aggressions, our epidermal lipids and other components form a natural protective barrier on the surface of the skin. However, when skin is repeatedly washed and exposed to a variety of exogenous stimuli, such as allergens, irritant solvents, ultraviolet rays, microorganisms, particles, and a series of stimuli, coupled with low resistance, the integrity of a sebum film and other epidermal structures formed by free fatty acids, triglycerides, waxes, and cholesterol in the stratum corneum will be damaged, eventually leading to impaired skin barrier function, which consequently causes skin diseases such as skin dryness, aging, skin sensitivity such as pigmentation atopic dermatitis, irritant dermatitis, and hormone-dependent dermatitis.

The skin barrier is a natural protective film of the skin, and generally refers to a physical or mechanical barrier structure of the epidermis, especially the stratum corneum, where epidermal keratinocytes are like "bricks" of a wall, while "lipids" (special lipids between keratinocytes, composed of ceramides, cholesterol, cholesterol esters, fatty acids, etc.) and "natural moisturizing factors" between corneocytes are like "mortar" of the wall, which tightly connect keratinocytes, limit water flow inside and outside the cells and between the cells, and ensure that the water is not lost. At the same time, there is a layer of hydrolipidic film covering the brick wall structure, which is mainly formed by emulsifying sebum secreted by sebaceous glands (containing about 57.5% of triglycerides and their hydrolysis products, 26% of wax ester, 12% of squalene, 3% of cholesterol ester and 1.5% of cholesterol), lipids (mainly ceramides) produced by the disintegration of keratinocytes, and sweat secreted by sweat glands. The hydrolipidic film and the brick wall structure together constitute a physical barrier of skin. It daily provides isolation and protection, regulates and controls the absorption of foreign substances through the skin, and prevents evaporation of moisture from the skin, thereby having functions of moisturizing and regulating an anti-inflammatory effect.

The reference doucument CN110384652A discloses a composition capable of lowering sensitivity and hormone dependency in skin care and a preparation method of the composition, and belongs to the technical field of skin care treatment. According to the implementation mode, the composition capable of lowering the sensitivity and the hormone dependency in skin care is characterized by being prepared from the following content of a centella asiatica extract, a calendula extract, a chamomile extract, a radix glycyrrhizae/radix scutellariae/opuntia dillenii haw extract, a bacillus/soybean fermentation product extract, human-like collagen, a bisabolol/ginger root extract, tocopheryl acetate, allantoin, ceramide, caprylhydroxamic acid, tocopherol, folic acid, panthenol, an emollient, a humectant, a bacteriostat and water.All the components of the composition influence each other and act synergistically, the skin is nourished, moisturized, improved and protected, and the skin problems such as skin allergy, eczematousdermatitis and hormone dependent dermatitis caused by multiple reasons can be solved; and the composition is free of toxicity and hormones and suitable for being used for a long term or a short term,and has large market application demands.

The reference document CN110013445A discloses a bionic sebum composition for skin barrier repair. The bionic sebum composition comprises the following components in parts by weight of 70-90 parts of limnanthes alba seed oil, 5-15 parts of squalane, 1-3 parts of phytosteryl oleate, 1-4 parts of ceramide, 1-3 parts of lecithin, 1-3 parts of cholesterol, 0.1-0.8 part of vitamin E and 0.1-0.7 part of isooctadecanol. The prepared namely the bionic sebum composition does not contain harmful chemical industry raw materials, have components close to skin components of human bodies, have the effects of complementing sebum and repairing damaged barriers of skin and is used for cosmetics or medicine products.

The reference document CN107440942A provides essential oil for softening and moisturizing skin. The essential oil has the functions of softening the skin, providing a barrier moisturizing function for the skin and brightening skin tone. The essential oil for softening and moisturizing the skin is prepared from the following components in parts by weight: 40 to 70 parts of squalane, 20 to 30 parts of simmondsia chinensis seed oil, 1 to 5 parts of argania spinosa kernel oil, 0.05 to 1 part of tocopherol, 0.05 to 1 part of ethylhexylglycerin, 0.0001 to 0.2 part of lemon peel oil and 0.0001 to 0.2 part of verbena oil.

The reference document CN106943537A provides an antibacterial composition and a moisturizing and repairing composition containing camellia seed oil. The antibacterial composition includes: an extract of artemisia apiacea, an extract of fructus litsea cubeba, an extract of pogostemon cablin, and an extract of lemongrass. The moisturizing and repairing composition includes the following steps of: camellia seed oil, ceramide 2, phytosterol oleate, the extract of artemisia apiacea, the extract of fructus litsea cubeba, the extract of pogostemon cablin, the extract of lemongrass, an extract of camellia flowers and an extract of camellia leaves. The moisturizing and repairing composition is advantaged in that through optimum ratio of physiologic lipids (ceramide, phytosterol and camellia seed oil), the composition is easy to connect with keratinocytes, so that a keratin barrier has excellent continuity and a "brick wall" structure is reinforced; being different from common moisturizing principles, the moisturizing and repairing composition is free of addition of any chemical moisture absorbing raw materials and will not absorb moisture from interior of skin at any humidity from the angle of pure oil moisturation, so that a problem of skin barrier repairing is solved fundamentally.

The reference document US2012/093896A1 describes the use of a nanodispersion, which comprises: a) a membrane-forming molecule, b) a co-emulsifier, c) a lipophilic component, and d) a water-soluble ingredient sensitive to oxidation in cosmetic end formulations, the nanodispersion being obtainable by (α) mixing the components (a), (b) and (c) until a homogeneous liquid is obtained (so-called nanodispersion pre-phase), and (&bgr;) adding the water or aqueous phase (discontinuous phase) which contains the water-soluble ingredient and optionally the non-aqueous polar organic solvent to the liquid obtained in step (α). Component (d) is preferably ascorbic acid or vitamin C.

The reference document CN110448486A discloses essential oil capable of repairing an impaired sebum membrane. The essential oil comprises helianthus annuus seed oil, camellia japonica seed oil, a carthamus tinctorius flowerextract, a gardenia florida extract, a chrysanthemum morifolium extract and a prunus mume extract. According to the essential oil, the helianthus annuus seed oil and the camellia japonica seed oil are selected as oily components, the carthamus tinctorius flower extract, the gardenia florida extract, the chrysanthemum morifolium extract and the prunus mume extract are taken as main active components, the impaired sebum membrane can be effectively repaired synergistically, the content of moisture of the skin is increased, and the skin allergy rate is lowered.

The reference document CN108743474A discloses an ointment like baby diaper rash cream and a preparation method thereof, and relates to the technical field of daily skin care products. The baby diaper rash cream comprises following components in percentage by weight: 5.000 to 20.000% of mixed wax, 7.000 to 70.000% of plant oil, 5.000 to 10.000% of plant grease, 0.001 to 7.000% of plant extract, and 0.25 to 1.500% of antioxidant. The provided baby diaper rash cream is mild and nonirritant, can partition the baby skin from diapers so as to protect the tender skin of babies from friction, and also has the functions ofkilling bacteria and eliminating inflammation.

The reference document CN109498475A provides a camellia seed oil-containing micro-emulsion mask liquid with a repair effect and a preparation method thereof. The mask liquid is prepared from 10 to 60 percent of polyol, 0.5to 10.0 percent of lecithin, 0.05 to 5.0 percent of camellia seed oil, 0.5 to 5.0 percent of camellia flower and camellia leaf extracts, 0.05 to 1.0 percent of sterols, 0.005 to 0.5 percent of a ceramide compound, 0.05 to 1.0 percent of a thickener and 35 to 85 percent of water. According to the camellia seed oil-containing micro-emulsion mask liquid with the repair effect, insoluble effect substances such as the camellia seed oil, the ceramide and the sterols are prepared into the micro-emulsion mask liquid by adopting the lecithin serving as an emulsifier and a specific component polyol serving as a solvent, so that the effect substances are laid on the face with a mask fabric in the form of micro-emulsion to quickly infiltrate into the skin to repair a skin barrier and achieve good anddurable moisturizing effects. The micro-emulsion mask liquid is high in stability and is not layered even after being subjected to a centrifugal test.

The reference document CN110859762A provides camellia extract-containing gel with an after-sun repair effect and a preparation method thereof. The gel is prepared from the following components in percentage by weight: 5 to20 percent of camellia extract inclusion; 10 to 40 percent of aloe leaf juice; 0.1 to 1 percent of asiaticoside; 0.1 to 1 percent of radix sophorae flavescentis extract; 0.1 to 1 percent of beta-glucan; 0.01 to 0.2 percent of dipotassium glycyrrhizinate; 0.01 to 0.1 percent of sodium hyaluronate; 0.1 to 1 percent of a thickening agent; 0.1 to 1 percent of a preservative; and the balanced of deionized water. The gel with the after-sun repair effect also comprises a pH regulator which is added to adjust the pH value to be neutral. According to the invention, a plurality of functional componentsare compounded according to an optimal ratio, so that the gel can effectively relieve skin inflammation, repair skin barrier, supplement water and preserve moisture, and can effectively relieve skindiscomfort caused by strong sunlight irradiation.

The reference document EP2133066A1 relates to a cosmetic or dermatological composition consisting of the following components: (i) a safe and effective amount of a mixture consisting of two ore more unsaturated C18 fatty acid esters of retinol and, optionally, one or more saturated C16-C18 fatty acid ester of retinol; (ii) a safe and effective amount of one or more tocotrienols; (iii) optionally, a safe and effective amount of one or more tocopherols; and (iv) one or more cosmetically or dermatologically acceptable carriers, wherein the components (i), (ii) and (iii) optionally include either non active components or non effective amounts of other components. The composition provides an improvement of the cosmetic appearance of the skin that is higher than expected due to a unexpected synergistic effect. It is also useful for the treatment of dermatological disorders. In conclusion, a healthy and intact epidermal barrier plays a crucial role in protecting people from the external environment. It can be used as a physical barrier to prevent the invasion of irritants, allergens, and other harmful chemicals, as well as an outlet for excess water, thus protecting the body from infection, irritation, and dehydration.

Therefore, it is necessary to prepare a biomimetic natural skin barrier repair composition and a formulation thereof, which not only improve skin elasticity, refine and smooth the skin, have a good repair effect, but do not damage the skin.

### Summary of the Invention

Aiming at the existing technology, an objective of the present invention is to provide a biomimetic and camellia extract-containing skin barrier repair composition and application. The biomimetic and camellia extract-containing skin barrier repair composition of the present invention has the effects of repairing the skin barrier, improving the skin elasticity, improving the skin fineness, and effectively improving the skin condition, and can be used as a safe and natural functional ingredient to be added to cosmetics. Therefore, the present invention also provides application of the above-mentioned biomimetic and camellia extract-containing skin barrier repair composition in products having the effects of repairing the skin barrier, improving the skin elasticity, and increasing the skin moisture content such as a cosmetic soothing oil and lotion.

The present invention achieves the above objectives by the following technical solutions.

The present invention provides a biomimetic and camellia extract-containing skin barrier repair composition, which consists of the following parts:
sebum film repair components: a squalene, a fatty acid, a triglyceride, and a cholesterol analog;
keratinocyte interstitial components: a free fatty acid, a ceramide, and a cholesterol analog; and
synergistic components: a soothing and anti-inflammatory ingredient, an anti-aging ingredient, a whitening ingredient, and a skin penetrant,
wherein in the sebum film repair components, the squalene includes squalane and a camellia extract, the fatty acid and the triglyceride are provided by one or more of the camellia extract, an oat kernel oil, and a camelina oil, and the cholesterol analog is a phytosterol;
in the keratinocyte interstitial components, the free fatty acid is provided by one or more of the camellia extract, the oat kernel oil, and the camelina oil, the ceramide is provided by one or more of ceramide 3 and the oat kernel oil, and the cholesterol analog is a phytosterol;
wherein the camellia extract includes a camellia seed oil, a camellia flower extract, and a camellia leaf extract, and in the synergistic components, the soothing and anti-inflammatory ingredient includes one or more of a botanical soothing combination comprising a combination of multiple extracts selected from a *Matricaria recutita* leaf extract, a *Centella asiatica* extract, a *Rosmarinus officinalis* leaf extract, a *Scutellaria baicalensis* root extract, a tea leaf extract, and a *Reynoutria japonica* root extract,
bisabolol, and a *Melaleuca alternifolia* leaf oil; the anti-aging ingredient includes a tocopherol or vitamin E, and a *Rosa rubiginosa* seed oil; the whitening ingredient is a *Glycyrrhiza glabra* root extract, and the skin penetrant is a *Melaleuca alternifolia* leaf oil.

The present invention also provides a biomimetic and camellia extract-containing skin barrier repair composition, which includes the following raw materials in percentage by weight:

| | |
|---|---|
| camellia extract | 10-90%, |
| squalane | 3-60%, |
| camelina oil | 2-20%, |
| *Rosa rubiginosa* seed oil | 1-50%, |
| oat kernel oil | 1-20%, |
| bisabolol | 0.1-2%, |
| phytosterol | 0.5-5%, |
| *Melaleuca alternifolia* leaf oil | 0.1-2%, |
| tocopherol or vitamin E | 0.1-2%, |
| botanical soothing combination | 0.1-5%, |
| fragrance or essential oil | 0.1-2%, |
| ceramide 3 | 0.01-1%, and |
| *Glycyrrhiza glabra* root extract | 0.01-1%. |

As an embodiment of the present invention, the composition includes the following raw materials in percentage by weight:

| | |
|---|---|
| camellia extract | 30-75%, |
| squalane | 4.5-35%, |
| camelina oil | 2.8-15%, |
| *Rosa rubiginosa* seed oil | 3.5-15%, |
| oat kernel oil | 4-10%, |
| bisabolol | 0.1-1%, |
| phytosterol | 0.5-3.6%, |
| *Melaleuca alternifolia* leaf oil | 0.1-1.2%, |
| tocopherol or vitamin E | 0.1-1.0%, |
| botanical soothing combination | 0.5-2.0%, |
| fragrance or essential oil | 0.1-1.2%, |
| ceramide 3 | 0.01-0.8%, and |
| *Glycyrrhiza glabra* root extract | 0.01-1%. |

As an embodiment of the present invention, the botanical soothing combination includes the following parts in percentage by weight:

| | |
|---|---|
| *Matricaria recutita* leaf extract | 5-30%, |
| *Centella asiatica* extract | 2-40%, |
| *Rosmarinus officinalis* leaf extract | 3-20%, |
| *Scutellaria baicalensis* root extract | 5-30%, |
| tea leaf extract | 1-20%, and |
| *Reynoutria japonica* root extract | 10-20%. |

As an embodiment of the present invention, in the camellia extract, a mass ratio of the camellia seed oil to the camellia flower extract to the camellia leaf extract is 72-99.5: 0.1-15: 0.1-15.

As an embodiment of the present invention, a weight ratio of the camellia extract to the squalane to the camelina oil to the oat kernel oil to the phytosterol to the ceramide 3 is 13-16: 1: 0.5-1.5: 0.5-1.0: 0.4-0.8: 0.05-0.15. As a preferred example of the present embodiment, a weight ratio of the camellia extract to the squalane to the camelina oil to the oat kernel oil to the phytosterol to the ceramide 3 is 14.6: 1: 1: 0.8: 0.6: 0.1.

The present invention also provides application of the biomimetic and camellia extract-containing skin barrier repair composition in preparation of a cosmetic.

As an embodiment of the present invention, the cosmetic is a cosmetic for repairing and/or preventing damage to the stratum corneum and damage to a sebum film of the skin.

Compared with the prior art, the present invention has the following beneficial effects:
1. on the basis of a large number of formulation tests and studies, and taking the human skin barrier as a standard, the skin barrier repair composition of the present invention biomimetically proportions keratinocyte interstitial components and sebum film repair components in a similar manner as in the human skin barrier structure, so as to safely simulate the components of the skin barrier and restore a complete and healthy epidermal barrier;
2. the raw materials used in the skin barrier repair composition of the present invention are all organic certified, safe and natural; the oat kernel oil contains ceramides, phospholipids, sterols, triglycerides, linoleic acid, and the like; the camellia extract contains squalene, unsaturated fatty acids, triglycerides, phytosterols, vitamin E, and the like; and the camelina oil is rich in unsaturated fatty acids, especially Omega 3 (ALA), which plays an important role in regulating the body's anti-inflammatory mechanism and tissue regeneration. On the basis of a large number of formulation tests and studies, the composition of present invention scientifically proportions a camellia extract, a camelina oil, an oat kernel oil, squalane, a phytosterol, and ceramide 3, so as to maximally simulate the components of the skin barrier, effectively improve the absorption and stability, and strengthen a barrier repair effect;
3. the composition of the present invention contains abundant skin barrier repair components in combination with synergistic components and soothing and anti-inflammatory, anti-aging and whitening ingredients, which enables the skin to be favorable for the existence of synergistic and mutually beneficial microbial flora, protects the skin from the invasion of harmful microorganisms, and has targeting and synergistic effects; and
4. the ingredients (such as the *Melaleuca alternifolia* leaf oil, the bisabolol, and the *Rosa rubiginosa* seed oil) in the synergistic components of the present invention can play a synergistic role in repairing the skin barrier while playing corresponding soothing and anti-inflammatory, anti-aging and whitening effects.

### Detailed Description of the Invention

The present invention will be described in details below with reference to specific examples.

A *Melaleuca alternifolia* leaf oil, an oat kernel oil, a camellia seed oil, a *Rosa rubiginosa* seed oil, camelina oil, a camellia flower extract, a camellia leaf extract, a *Glycyrrhiza glabra* root extract, a *Matricaria recutita* leaf extract, *a Centella asiatica* extract, a *Rosmarinus officinalis* leaf extract, a *Scutellaria baicalensis* root extract, a tea leaf extract, and a *Reynoutria japonica* root extract involved in the present invention can be purchased from the market, and can also be obtained by conventional extraction methods.

### Example 1

The present example provides a camellia extract-containing skin barrier repair composition, including the following parts:

| | |
|---|---|
| camellia extract | 73%, |
| squalane | 5%, |
| camelina oil | 5%, |
| *Rosa rubiginosa* seed oil | 4%, |
| oat kernel oil | 4%, |
| bisabolol | 1%, |
| phytosterol | 3%, |
| *Melaleuca alternifolia* leaf oil | 1%, |
| tocopherol | 1%, |
| botanical soothing combination | 1%, |
| fragrance | 1%, |
| ceramide 3 | 0.5%, and |
| *Glycyrrhiza glabra* root extract | 0.5%. |

Among them, the camellia extract: the trade name of Qingxuan Cui, purchased from Anji Linqingxuan Agricultural Technology Co. Ltd.;
a preparation method of the botanical soothing combination: 1) a *Matricaria recutita* leaf extract, a *Centella asiatica* extract, a *Rosmarinus officinalis* leaf extract, a *Scutellaria baicalensis* root extract, a tea leaf extract, and a *Reynoutria japonica* root extract were purchased from the market; 2) the *Centella asiatica* extract, the *Matricaria recutita* leaf extract, the *Rosmarinus officinalis* leaf extract, the *Reynoutria japonica* root extract, the *Scutellaria baicalensis* root extract, and the tea leaf extract were added in a ratio of 8: 4: 4: 2: 1: 1 successively at a temperature of 25-35°C, mixed uniformly and filtered to obtain the botanical soothing combination.

For convenience of comparison, the camellia extract and the botanical soothing combination involved in the remaining examples and comparative examples are the same as those in the above example. In addition, in the present example, the fragrance may be replaced with an essential oil and the tocopherol may be replaced with vitamin E.

### Example 2

The present example provides a camellia extract-containing skin barrier repair composition, including the following parts:

| | |
|---|---|
| camellia extract | 72.6%, |
| squalane | 5.6%, |
| camelina oil | 2.8%, |
| *Rosa rubiginosa* seed oil | 3.5%, |
| oat kernel oil | 5.6%, |
| bisabolol | 0.8%, |
| phytosterol | 2.2%, |
| *Melaleuca alternifolia* leaf oil | 1.2%, |
| tocopherol | 0.8%, |
| botanical soothing combination | 2.0%, |
| fragrance | 1.2%, |
| ceramide 3 | 0.8%, and |
| *Glycyrrhiza glabra* root extract | 0.9%. |

### Example 3

The present example provides a camellia extract-containing skin barrier repair composition, including the following parts:

| | |
|---|---|
| camellia extract | 72.7%, |
| squalane | 4.5%, |
| camelina oil | 6.8%, |
| *Rosa rubiginosa* seed oil | 5%, |
| oat kernel oil | 2.3%, |
| bisabolol | 1%, |
| phytosterol | 3.6%, |
| *Melaleuca alternifolia* leaf oil | 0.6%, |
| tocopherol | 1%, |
| botanical soothing combination | 0.8%, |
| fragrance | 0.5%, |
| ceramide 3 | 0.2%, and |
| *Glycyrrhiza glabra* root extract | 1%. |

### Example 4

The present example provides a camellia extract-containing skin barrier repair composition, including the following parts:

| | |
|---|---|
| camellia extract | 60%, |
| squalane | 10%, |
| camelina oil | 8%, |
| *Rosa rubiginosa* seed oil | 6%, |
| oat kernel oil | 4%, |
| bisabolol | 0.8%, |
| phytosterol | 4.5%, |
| *Melaleuca alternifolia* leaf oil | 1%, |
| tocopherol | 1%, |
| botanical soothing combination | 3.2%, |
| fragrance | 0.5%, |
| ceramide 3 | 0.5%, and |
| *Glycyrrhiza glabra* root extract | 0.5%. |

### Example 5

The present example provides a camellia extract-containing skin barrier repair composition, including the following parts:

| | |
|---|---|
| camellia extract | 77.5%, |
| squalane | 5.3%, |
| camelina oil | 5.3%, |
| *Rosa rubiginosa* seed oil | 1%, |
| oat kernel oil | 4.3%, |
| bisabolol | 0.1%, |
| phytosterol | 5%, |
| *Melaleuca alternifolia* leaf oil | 0.1%, |
| tocopherol | 0.1%, |
| botanical soothing combination | 0.1%, |
| fragrance | 0.1%, |
| ceramide 3 | 1%, and |
| *Glycyrrhiza glabra* root extract | 0.1%. |

### Example 6

The present example provides a camellia extract-containing skin barrier repair composition, including the following parts:

| | |
|---|---|
| camellia extract | 10%, |
| squalane | 60%, |
| camelina oil | 5%, |
| *Rosa rubiginosa* seed oil | 20%, |
| oat kernel oil | 3%, |
| bisabolol | 0.5%, |
| phytosterol | 0.5%, |
| *Melaleuca alternifolia* leaf oil | 0.1%, |
| tocopherol | 0.1%, |
| botanical soothing combination | 0.1%, |
| fragrance | 0.1%, |
| ceramide 3 | 0.5%, and |
| *Glycyrrhiza glabra* root extract | 0.1%. |

### Example 7

The present example provides a camellia extract-containing skin barrier repair composition, including the following parts:

| | |
|---|---|
| camellia extract | 60%, |
| squalane | 3%, |
| camelina oil | 20%, |
| *Rosa rubiginosa* seed oil | 5%, |
| oat kernel oil | 10%, |
| bisabolol | 0.5%, |
| phytosterol | 0.5%, |
| *Melaleuca alternifolia* leaf oil | 0.1%, |
| tocopherol | 0.1%, |
| botanical soothing combination | 0.1%, |
| fragrance | 0.1%, |
| ceramide 3 | 0.5%, and |
| *Glycyrrhiza glabra* root extract | 0.1%. |

### Comparative Example 1

The comparative example provides a camellia extract-containing skin barrier repair composition, including the following parts:

| | |
|---|---|
| *Hippophae rhamnoides* seed oil | 60%, |
| squalane | 3%, |
| camelina oil | 20%, |
| *Rosa rubiginosa* seed oil | 5%, |
| oat kernel oil | 10%, |
| bisabolol | 0.5%, |
| phytosterol | 0.5%, |
| *Melaleuca alternifolia* leaf oil | 0.1%, |
| tocopherol | 0.1%, |
| botanical soothing combination | 0.1%, |
| fragrance | 0.1%, |
| ceramide 3 | 0.5%, and |
| *Glycyrrhiza glabra* root extract | 0.1%. |

### Comparative Example 2

The comparative example provides a camellia extract-containing skin barrier repair composition, including the following parts:

| | |
|---|---|
| camellia extract | 60%, |
| squalane | 3%, |
| camelina oil | 20%, |
| *Rosa rubiginosa* seed oil | 5%, |
| wheat germ oil | 10%, |
| bisabolol | 0.5%, |
| phytosterol | 0.5%, |
| *Melaleuca alternifolia* leaf oil | 0.1%, |
| tocopherol | 0.1%, |
| botanical soothing combination | 0.1%, |
| fragrance | 0.1%, |
| ceramide 3 | 0.5%, and |
| *Glycyrrhiza glabra* root extract | 0.1%. |

### Comparative Example 3

The comparative example provides a camellia extract-containing skin barrier repair composition, including the following parts:

| | |
|---|---|
| camellia extract | 60%, |
| squalane | 3%, |
| *Gossypium arboretum* seed oil | 20%, |
| *Rosa rubiginosa* seed oil | 5%, |
| oat kernel oil | 10%, |
| bisabolol | 0.5%, |
| phytosterol | 0.5%, |
| *Melaleuca alternifolia* leaf oil | 0.1%, |
| tocopherol | 0.1%, |
| botanical soothing combination | 0.1%, |
| fragrance | 0.1%, |
| ceramide 3 | 0.5%, and |
| *Glycyrrhiza glabra* root extract | 0.1%. |

### Comparative Example 4

The comparative example provides a camellia extract-containing skin barrier repair composition, including the following parts:

| | |
|---|---|
| *Hippophae rhamnoides* seed oil | 60%, |
| squalane | 3%, |
| camelina oil | 20%, |
| *Rosa rubiginosa* seed oil | 5%, |
| wheat germ oil | 10%, |
| bisabolol | 0.5%, |
| phytosterol | 0.5%, |
| *Melaleuca alternifolia* leaf oil | 0.1%, |
| tocopherol | 0.1%, |
| botanical soothing combination | 0.1%, |
| fragrance | 0.1%, |
| ceramide 3 | 0.5%, and |
| *Glycyrrhiza glabra* root extract | 0.1%. |

### Comparative Example 5

The comparative example provides a camellia extract-containing skin barrier repair composition, including the following parts:

| | |
|---|---|
| camellia extract | 28.1%, |
| squalane | 60%, |
| camelina oil | 5%, |
| oat kernel oil | 3%, |
| bisabolol | 0.5%, |
| phytosterol | 0.5%, |
| *Melaleuca alternifolia* leaf oil | 0.1%, |
| tocopherol | 2%, |
| botanical soothing combination | 0.1%, |
| fragrance | 0.1%, |
| ceramide 3 | 0.5%, and |
| *Glycyrrhiza glabra* root extract | 0.1%. |

### Comparative Example 6

The comparative example provides a camellia extract-containing skin barrier repair composition, including the following parts:

| | |
|---|---|
| camellia extract | 80.7%, |
| squalane | 5.5%, |
| camelina oil | 5.5%, |
| oat kernel oil | 4.4%, |
| phytosterol | 3.3%, and |
| ceramide 3 | 0.6%. |

### Comparative Example 7

The comparative example provides a camellia extract-containing skin barrier repair composition, including the following parts:

| | |
|---|---|
| *Limnanthes alba* seed oil | 83.37%, |
| squalane | 7.94%, |
| phytosterol oleate | 1.49%, |
| ceramide 3 | 1.79%, |
| ceramide 4 | 1.09%, |
| lecithin | 2.18%, |
| cholesterol | 1.49%, |
| vitamin E | 0.5%, and |
| isostearyl alcohol | 0.15%. |

The *Limnanthes alba* seed oil, the squalane, the phytosterol oleate, the ceramide 3, and the ceramide 4 were mixed, and heated at 70°C until the materials were completely dissolved to obtain a mixture A;
the lecithin, the cholesterol, and the vitamin E were mixed, and heated at 85°C until the materials were completely dissolved to obtain a mixture B; and
the mixture A and the mixture B were mixed and stirred at 95°C, cooled down to 50°C, added with the isostearyl alcohol, stirred until uniform, and cooled down to 20°C.

### Comparative Example 8

The comparative example provides a camellia extract-containing skin barrier repair composition, including the following parts:

| | |
|---|---|
| hydrogenated lecithin | 1.5%, |
| phytosterol | 2%, |
| olive-derived squalane | 3%, |
| C8-12 acid triglyceride | 2.3%, |
| spent grain wax | 2%, |
| hydrolyzed pearl | 2.5%, |
| *Butyrospermum parkii* extract | 2.5%, |
| *Argania spinosa* kernel oil | 0.5%, |
| hyaluronic acid | 0.5%, |
| vitamin E | 0.8%, |
| thickener | 0.1%, |
| emulsifier | 0.2%, |
| preservative | 0.05%, and |
| deionized water | 82.05%. |

The hydrogenated lecithin, the phytosterol, the olive-derived squalane, the *Argania spinosa* kernel oil, and the hyaluronic acid were uniformly dispersed with 1,3-propanediol and glycerol, then the mixture and the C8-12 acid triglyceride were added into water together, and heated to 83°C to prepare a mixture A;
the *Butyrospermum parkii* extract, the spent grain wax, the vitamin E, and the hydrolyzed pearl were mixed and heated to 83°C for dissolution to prepare a mixture B; and
the mixture B was added to the mixture A at a stirring speed of 43 rpm, and the mixture was stirred and homogenized, added with sodium hydroxide, stirred uniformly, and discharged.

### Efficacy verification 1: safety Test

The skin barrier repair compositions prepared in all of the above Examples and Comparative Examples were subjected to human safety patch tests according to a human skin patch test method described in the "Safety and Technical Standards for Cosmetics 2015". A skin closure patch test method is as follows: 30 persons aged 18-60 years old were selected, and a qualified patch test kit with an area of no more than 50 mm² and a depth of about 1 mm was selected. 0.020 mL of the skin barrier repair composition prepared in the above Examples and Comparative Examples was placed into a chamber of the patch test kit, and a control well was a blank control (without any substance). The patch test kit with the skin barrier repair composition was applied to a curved side of a forearm of the subject with a hypoallergenic tape, and was uniformly applied to the skin with gentle pressure by the palm for 24 h. Skin reactions were observed at 30 min (after indentation disappeared), 24 h and 48 h after removing the patch test kit containing the skin barrier repair composition according to the criteria in Table 1, and observation results were recorded. Patch test results for the skin barrier repair compositions are shown in Table 2.

Test results show that all the skin barrier repair compositions prepared in Examples and Comparative Examples pass the human patch safety test.

**Table 1 Grading criteria for skin reactions in skin closure patch test**

| Reaction degree | Scoring grade | Skin reactions |
|---|---|---|
| | 0 | Negative reaction |
| ± | 1 | Suspicious reaction with only faint erythema |
| + | 2 | Weak positive reaction (erythema reaction); erythema, infiltration, edema, and papules possible |
| ++ | 3 | Strong positive reaction (herpes reaction); erythema, infiltration, edema, papules, and herpes; possible reaction beyond the test area: |
| +++ | 4 | Very strong positive reaction (confluent herpes reaction); obvious erythema, severe infiltration, edema, and confluent herpes; possible reaction beyond test area |

**Table 2 Human Safety Test Results**

| Sample No. | 30 min | 24 h | 48 h | Summarized results |
|---|---|---|---|---|
| Example 1 | 0 | 0 | 0 | No positive reaction in 30 persons |
| Example 2 | 0 | 0 | 0 | No positive reaction in 30 persons |
| Example 3 | 0 | 0 | 0 | No positive reaction in 30 persons |
| Example 4 | 0 | 0 | 0 | No positive reaction in 30 persons |
| Example 5 | 0 | 0 | 0 | No positive reaction in 30 persons |
| Example 6 | 0 | 0 | 0 | No positive reaction in 30 persons |
| Example 7 | 0 | 0 | 0 | No positive reaction in 30 persons |
| Comparative Example 1 | 0 | 0 | 0 | No positive reaction in 30 persons |
| Comparative Example 2 | 0 | 0 | 0 | No positive reaction in 30 persons |
| Comparative Example 3 | 0 | 0 | 0 | No positive reaction in 30 persons |
| Comparative Example 4 | 0 | 0 | 0 | No positive reaction in 30 persons |
| Comparative Example 5 | 0 | 0 | 0 | No positive reaction in 30 persons |
| Comparative Example 6 | 0 | 0 | 0 | No positive reaction in 30 persons |
| Comparative Example 7 | 0 | 0 | 0 | No positive reaction in 30 persons |
| Comparative Example 8 | 0 | 0 | 0 | No positive reaction in 30 persons |

### Efficacy verification 2: repairing the skin barrier - transepidermal water loss rate

The skin barrier repair compositions prepared in all of the above Examples and Comparative Examples were tested for transepidermal water loss from cheeks using a transepidermal water loss meter (Tewameter) according to the following test methods: 30 healthy women aged 30±2 years old were selected for each group of diluted skin barrier repair composition samples. The samples were smeared on the cheeks. The transepidermal water loss rate of the skin was tested by the meter before sample application and after four weeks of sample application and was tested 3 times in parallel, an average value was taken, and a TEWL value was recorded.

Where the rate of change is the rate of change relative to that before use, and is calculated as follows:$Δ \left(difference value\right) after four weeks = {T}_{4} - {T}_{0}$$\begin{matrix}the rate of change of transepidermal water loss rate after four weeks of use relative to that before use \left(%\right) \\ = \frac{{\sum }_{i = T}^{i = N} \left({T}_{4}-{T}_{0}\right) / {T}_{0}}{N} \times 100\end{matrix}$

Where, T₀ - a TEWL value before cosmetic application in the test area.
T₄ - a TEWL value after four weeks of cosmetic application in the test area.
N - number of subjects.

Test results are shown in Table 3. The lower the TEWL, the better a skin barrier repair effect. The comparative examples are significantly different from the examples, while the compositions of the present invention repair the skin barrier better.

**Table 3**

| No. | TEWL value (average value) | | △ Difference value | Rate of change (%) |
|---|---|---|---|---|
| | 0 week | 4 weeks | | |
| Example 1 | 22.05 | 13.54 | -8.51 | -38.72% |
| Example 2 | 23.43 | 14.49 | -8.94 | -38.14% |
| Example 3 | 22.35 | 13.87 | -8.48 | -37.92% |
| Example 4 | 21.17 | 13.72 | -7.45 | -35.23% |
| Example 5 | 21.98 | 14.02 | -7.96 | -36.19% |
| Example 6 | 20.95 | 13.67 | -7.28 | -34.73% |
| Example 7 | 22.48 | 14.81 | -7.67 | -34.22% |
| Comparative Example 1 | 22.41 | 14.82 | -7.59 | -33.82% |
| Comparative Example 2 | 21.87 | 14.84 | -7.03 | -32.11% |
| Comparative Example 3 | 22.35 | 15.26 | -7.09 | -31.71% |
| Comparative Example 4 | 22.21 | 15.29 | -6.92 | -31.13% |
| Comparative Example 5 | 21.65 | 14.48 | -7.17 | -33.14% |
| Comparative Example 6 | 23.01 | 15.19 | -7.82 | -33.87% |
| Comparative Example 7 | 22.36 | 14.72 | -7.64 | -34.08% |
| Comparative Example 8 | 22.91 | 15.13 | -7.78 | -33.76% |

### Efficacy verification 3: repairing the skin barrier - stratum corneum moisture content

The skin barrier repair compositions prepared in all of the above Examples and Comparative Examples were tested for stratum corneum moisture content of cheek skin using a skin stratum corneum moisture content meter (Corneometer) according to the following test methods: 30 healthy women aged 30±2 years old were selected for each group of diluted skin barrier repair composition samples. The samples were smeared on the cheeks. The stratum corneum moisture content of cheek skin was tested by the meter before sample application and after four weeks of sample application and was tested 3 times in parallel, an average value was taken, and a stratum corneum moisture content value was recorded.

Where the rate of change is the rate of change relative to that before use, and is calculated as follows:$Δ \left(difference value\right) after four weeks = {T}_{4} - {T}_{0}$ $\begin{matrix}the rate of change of stratum corneum moisture content after four weeks of use relative to that before use \left(%\right) \\ = \frac{{\sum }_{i = T}^{i = N} \left({T}_{4}-{T}_{0}\right) / {T}_{0}}{N} \times 100\end{matrix}$

Where, T₀ - a stratum corneum moisture content value before cosmetic application in the test area.
T₄ - a stratum corneum moisture content value after four weeks of cosmetic application in the test area.
N - number of subjects.

Test results are shown in Table 4. The higher the skin moisture content, the better the skin moisture retention and the better the skin barrier function. The comparative examples are significantly different from the examples, while the skin barrier repair compositions of the present invention have a better moisturizing effect.

**Table 4**

| No. | Stratum corneum moisture content value (average value) | | △ difference value | Rate of change (%) |
|---|---|---|---|---|
| | 0 week | 4 weeks | | |
| Example 1 | 43.21 | 58.39 | 15.18 | 35.13% |
| Example 2 | 44.88 | 59.30 | 14.42 | 32.13% |
| Example 3 | 43.56 | 57.53 | 13.97 | 32.05% |
| Example 4 | 42.12 | 54.64 | 12.52 | 29.87% |
| Example 5 | 40.78 | 53.43 | 12.65 | 30.78% |
| Example 6 | 41.25 | 53.12 | 11.87 | 28.67% |
| Example 7 | 40.21 | 51.30 | 11.09 | 27.54% |
| Comparative Example 1 | 43.38 | 53.97 | 10.59 | 24.38% |
| Comparative Example 2 | 44.44 | 55.11 | 10.67 | 23.97% |
| Comparative Example 3 | 42.48 | 52.06 | 9.58 | 22.47% |
| Comparative Example 4 | 43.56 | 53.15 | 9.59 | 21.98% |
| Comparative Example 5 | 42.98 | 53.35 | 10.37 | 24.11% |
| Comparative Example 6 | 41.78 | 52.60 | 10.82 | 25.84% |
| Comparative Example 7 | 42.34 | 53.63 | 11.29 | 26.62% |
| Comparative Example 8 | 41.62 | 51.73 | 10.11 | 24.21% |

### Efficacy Verification 4: skin elasticity

The skin barrier repair compositions prepared in all of the above Examples and Comparative Examples were tested for human skin elasticity using a skin elasticity tester (Cutometer) according to the following test methods: 30 healthy women aged 30±2 years old were selected for each group of diluted skin barrier repair composition samples. The samples were smeared on the cheeks. The skin elasticity was tested by the tester before sample application and after four weeks of sample application and was tested 3 times in parallel, an average value was taken, and an R2 value was recorded.

Where the rate of change is the rate of change relative to that before use, and is calculated as follows:$Δ \left(difference value\right) after four weeks = {T}_{4} - {T}_{0}$$\begin{matrix}the rate of change of skin elasticity after four weeks of use relative to that before use \left(%\right) \\ = \frac{{\sum }_{i = T}^{i = N} \left({T}_{4}-{T}_{0}\right) / {T}_{0}}{N} \times 100\end{matrix}$

Where, T₀ - an R2 value before cosmetic application in the test area.
T₄ - an R2 value after four weeks of cosmetic application in the test area.
N - number of subjects.

Test results are shown in Table 5. The closer the skin elasticity R2 value is to 1, the better the skin elasticity. The higher the rate of change of the R2 value, the better the effect of the sample on improving skin elasticity. The comparative examples are significantly different from the examples, while the skin barrier repair compositions of the present invention have a better effect in improving skin elasticity.

**Table 5**

| No. | R2 value (average value) | | Δ Difference value | Rate of change (%) |
|---|---|---|---|---|
| | 0 week | 4 weeks | | |
| Example 1 | 0.6154 | 0.6966 | 0.0812 | 13.21% |
| Example 2 | 0.6214 | 0.7015 | 0.0801 | 12.87% |
| Example 3 | 0.6333 | 0.7107 | 0.0774 | 12.14% |
| Example 4 | 0.6992 | 0.7783 | 0.0791 | 11.14% |
| Example 5 | 0.6541 | 0.7320 | 0.0779 | 11.85% |
| Example 6 | 0.6094 | 0.6735 | 0.0641 | 10.87% |
| Example 7 | 0.5893 | 0.6501 | 0.0608 | 10.22% |
| Comparative Example 1 | 0.6372 | 0.7010 | 0.0638 | 9.47% |
| Comparative Example 2 | 0.6191 | 0.6745 | 0.0554 | 8.91% |
| Comparative Example 3 | 0.5741 | 0.6216 | 0.0475 | 8.21% |
| Comparative Example 4 | 0.6712 | 0.7244 | 0.0532 | 7.81% |
| Comparative Example 5 | 0.5923 | 0.6461 | 0.0538 | 9.05% |
| Comparative Example 6 | 0.6291 | 0.6914 | 0.0623 | 9.87% |
| Comparative Example 7 | 0.6287 | 0.6919 | 0.0632 | 10.05% |
| Comparative Example 8 | 0.6122 | 0.6730 | 0.0608 | 9.92% |

### Efficacy Verification 5: refining and smoothing skin - skin roughness

The camellia extract-containing skin elasticity repair compositions prepared in all of the above Examples and Comparative Examples were tested for human skin roughness using VisioScan VC98 USB (Courage & Khazaka) and corresponding software was applied to analyze an arithmetic mean skin roughness SEr value. The test method was as follows: 30 healthy women aged 30±2 years old were selected for each group of diluted camellia extract-containing restorative and firming composition samples. The samples were smeared on the cheeks. The skin roughness was tested by the tester before sample application and after four weeks of sample application and was tested 3 times in parallel, an average value was taken, and an SEr value was recorded.

Where the rate of change is the rate of change relative to that before use, and is calculated as follows:$Δ \left(difference value\right) after four weeks = {T}_{4} - {T}_{0}$$\begin{matrix}the rate of change of skin roughness after four weeks of use relative to that before use \left(%\right) \\ = \frac{{\sum }_{i = T}^{i = N} \left({M}_{4}-{M}_{0}\right) / {M}_{0}}{N} \times 100\end{matrix}$

Where, M₀ - an SEr value before cosmetic application in the test area.
M₄ - an SEr value after four weeks of cosmetic application in the test area.
N - number of subjects.

Test results are shown in Table 6. The SEr value characterizes the arithmetic mean skin roughness. The higher the value, the lower the skin roughness and the finer and smoother the skin. The comparative examples are significantly different from the example 1, while the skin barrier repair compositions of the present invention have a better refining and smoothing effect on the skin.

**Table 6**

| No. | SEr value (average value) | | Δ Difference value | Rate of change (%) |
|---|---|---|---|---|
| | 0 week | 4 weeks | | |
| Example 1 | 3.87 | 4.44 | 0.57 | 14.01% |
| Example 2 | 4.09 | 4.64 | 0.55 | 13.74% |
| Example 3 | 3.45 | 3.91 | 0.46 | 13.28% |
| Example 4 | 4.21 | 4.72 | 0.51 | 11.98% |
| Example 5 | 3.98 | 4.49 | 0.51 | 12.87% |
| Example 6 | 3.37 | 3.75 | 0.38 | 11.34% |
| Example 7 | 3.21 | 3.57 | 0.36 | 11.02% |
| Comparative Example 1 | 3.64 | 4.03 | 0.39 | 10.57% |
| Comparative Example 2 | 3.54 | 3.88 | 0.34 | 9.58% |
| Comparative Example 3 | 4.19 | 4.58 | 0.39 | 9.31% |
| Comparative Example 4 | 4.07 | 4.44 | 0.37 | 9.07% |
| Comparative Example 5 | 3.79 | 4.17 | 0.38 | 10.01% |
| Comparative Example 6 | 4.35 | 4.82 | 0.47 | 10.87% |
| Comparative Example 7 | 4.25 | 4.72 | 0.47 | 10.97% |
| Comparative Example 8 | 3.66 | 4.06 | 0.40 | 10.91% |

### Efficacy Verification 6: refining and smoothing skin - skin smoothness

The camellia extract-containing skin barrier repair compositions prepared in all of the above Examples and Comparative Examples were tested for human skin smoothness using VisioScan VC98 USB (Courage & Khazaka) and corresponding software was applied to analyze an arithmetic mean skin smoothness SEsm value. The test method was as follows: 30 healthy women aged 30±2 years old were selected for each group of diluted camellia extract-containing skin barrier repair composition samples. The samples were smeared on the cheeks. The skin smoothness was tested by the tester before sample application and after four weeks of sample application and was tested 3 times in parallel, an average value was taken, and an SEsm value was recorded.

Where the rate of change is the rate of change relative to that before use, and is calculated as follows: $Δ \left(difference value\right) after four weeks = {M}_{4} - {M}_{0}$$\begin{matrix}the rate of change of skin smoothness after four weeks of use relative to that before use \left(%\right) \\ = \frac{{\sum }_{i = T}^{i = N} \left({M}_{4}-{M}_{0}\right) / {M}_{0}}{N} \times 100\end{matrix}$

Where, M₀ - an SEsm value before cosmetic application in the test area.
M₄ - an SEsm value after four weeks of cosmetic application in the test area.
N - number of subjects.

Test results are shown in Table 4. The SEsm value characterizes the arithmetic mean skin smoothness. The lower the value, the higher the skin smoothness, and the higher the rate of change of SEsm value, the finer and smoother the skin is after sample application. The comparative examples are significantly different from the examples, while the skin barrier repair compositions of the present invention have a better skin refining and smoothing effect on the skin.

**Table 4**

| No. | SEsm value (average value) | | Δ Difference value | Rate of change (%) |
|---|---|---|---|---|
| | 0 week | 4 weeks | | |
| Example 1 | 217.56 | 199.01 | -18.55 | -8.52% |
| Example 2 | 209.52 | 192.31 | -17.22 | -8.21% |
| Example 3 | 250.12 | 230.35 | -19.77 | -7.89% |
| Example 4 | 222.54 | 206.91 | -15.63 | -7.01% |
| Example 5 | 214.63 | 199.15 | -15.48 | -7.21% |
| Example 6 | 233.47 | 217.40 | -16.07 | -6.87% |
| Example 7 | 201.66 | 187.85 | -13.81 | -6.82% |
| Comparative Example 1 | 221.97 | 208.05 | -13.92 | -6.25% |
| Comparative Example 2 | 234.67 | 220.61 | -14.06 | -5.98% |
| Comparative Example 3 | 254.67 | 239.75 | -14.92 | -5.84% |
| Comparative Example 4 | 231.41 | 218.10 | -13.31 | -5.74% |
| Comparative Example 5 | 218.74 | 205.35 | -13.39 | -6.11% |
| Comparative Example 6 | 211.54 | 197.92 | -13.62 | -6.45% |
| Comparative Example 7 | 222.21 | 207.26 | -14.95 | -6.71% |
| Comparative Example 8 | 215.37 | 201.25 | -14.12 | -6.54% |

### Application Example

This application example provides an application formulation and process of a skin barrier repair composition in camellia soothing oil, and the specific formulation is as follows:

| **No.** | **Standard Chinese name** | Content (%) |
|---|---|---|
| 1 | skin barrier repair composition | 90.0 |
| 2 | Caprylic/capric triglyceride | 3.0 |
| 3 | C15-19 alkyl | 1 |
| 4 | Dicaprylyl ether | 4 |
| 5 | Coco-caprylate/caprate | 2 |
| Total | | 100 |

Process flow: 1) the above materials are sequentially added into a stirring pot and stirred until uniform; and
2) the materials are filtered to obtain a camellia soothing oil.

The specific examples of the present invention are described. It should be understood that the present invention is not limited to the specific implementations described above, and those skilled in the art may make various transformations and modifications within the scope of the claims, which do not affect the substance of the present invention.

## Claims

1. A biomimetic and camellia extract-containing skin barrier repair composition, **characterized in that** the composition consists of the following parts:
sebum film repair components: a squalene, a fatty acid, a triglyceride, and a cholesterol analog;
keratinocyte interstitial components: a free fatty acid, a ceramide, and a cholesterol analog; and
synergistic components: a soothing and anti-inflammatory ingredient, an anti-aging ingredient, a whitening ingredient, and a skin penetrant,
wherein in the sebum film repair components, the squalene comprises squalane and a camellia extract, the fatty acid and the triglyceride are provided by one or more of the camellia extract, an oat kernel oil, and a camelina oil, and the cholesterol analog is a phytosterol;
in the keratinocyte interstitial components, the free fatty acid is provided by one or more of the camellia extract, the oat kernel oil, and the camelina oil, the ceramide is provided by one or more of ceramide 3 and the oat kernel oil, and the cholesterol analog is a phytosterol;
wherein the camellia extract comprises a camellia seed oil, a camellia flower extract and a camellia leaf extract:
and in the synergistic components, the soothing and anti-inflammatory ingredient comprises one or more of a botanical soothing combination comprising a combination of multiple extracts selected from a Matricaria recutita leaf extract,a Centella asiatica extract, a Rosmarinus officinalis leaf extract, a Scutellaria baicalensis root extract, a tea leaf extract and a Revnoutria japonica root extract, bisabolol, and a *Melaleuca alternifolia* leaf oil; the anti-aging ingredient comprises a tocopherol or vitamin E, and a *Rosa rubiginosa* seed oil; the whitening ingredient is a *Glycyrrhiza glabra* root extract, and the skin penetrant is a *Melaleuca alternifolia* leaf oil.

2. A biomimetic and camellia extract-containing skin barrier repair composition, according to claim 1, **characterized in that** the composition comprises the following raw materials in percentage by weight:
| | |
|---|---|
| camellia extract | 10-90%, |
| squalane | 3-60%, |
| camelina oil | 2-20%, |
| *Rosa rubiginosa* seed oil | 1-50%, |
| oat kernel oil | 1-20%, |
| bisabolol | 0.1-2%, |
| phytosterol | 0.5-5%, |
| *Melaleuca alternifolia* leaf oil | 0.1-2%, |
| tocopherol or vitamin E | 0.1-2%, |
| botanical soothing combination | 0.1-5%, |
| fragrance or essential oil | 0.1-2%, |
| ceramide 3 | 001-1%, and |
| *Glycyrrhiza glabra* root extract | 0.01-1%. |

3. The biomimetic and camellia extract-containing skin barrier repair composition according to claim 2, **characterized in that** the botanical soothing combination comprises the following parts in percentage by weight:
| | |
|---|---|
| *Matricaria recutita* leaf extract | 5-30%, |
| *Centella asiatica* extract | 2-40%, |
| *Rosmarinus officinalis* leaf extract | 3-20%, |
| *Scutellaria baicalensis* root extract | 5-30%, |
| tea leaf extract | 1-20%, and |
| *Reynoutria japonica* root extract | 10-20%. |

4. The biomimetic and camellia extract-containing skin barrier repair composition according to claim 2, **characterized in that** in the camellia extract, a mass ratio of the camellia seed oil to the camellia flower extract to the camellia leaf extract is 72-99.5: 0.1-15: 0.1-15.

5. The biomimetic and camellia extract-containing skin barrier repair composition according to claim 2, **characterized in that** a weight ratio of the camellia extract to the squalane to the camelina oil to the oat kernel oil to the phytosterol to the ceramide 3 is 13-16: 1: 0.5-1.5: 0.5-1.0: 0.4-0.8: 0.05-0.15.

6. The biomimetic and camellia extract-containing skin barrier repair composition according to claim 2, **characterized in that** the composition comprises the following raw materials in percentage by weight:
| | |
|---|---|
| camellia extract | 30-75%, |
| squalane | 4.5-35%, |
| camelina oil | 2.8-15%, |
| *Rosa rubiginosa* seed oil | 3.5-15%, |
| oat kernel oil | 4-10%, |
| bisabolol | 0.1-1%, |
| phytosterol | 0.5-3.6%, |
| *Melaleuca alternifolia* leaf oil | 0.1-1.2%, |
| tocopherol or vitamin E | 0.1-1.0%, |
| botanical soothing combination | 0.5-2.0%, |
| fragrance or essential oil | 0.1-1.2%, |
| ceramide 3 | 0.01-0.8%, and |
| *Glycyrrhiza glabra* root extract | 0.01-1%. |

7. Use of the biomimetic and camellia extract-containing skin barrier repair composition according to claim 1 or 2 as a functional raw material in preparation of a cosmetic.

8. The use according to claim 7, **characterized in that** the cosmetic is a cosmetic for repairing and/or preventing damage to the stratum corneum and damage to a sebum film of the skin.

## Patentansprüche

1. Eine biomimetische und Camellia-extrakthaltige Hautbarrierereparaturzusammensetzung, **dadurch gekennzeichnet, dass** die Zusammensetzung aus den folgenden Teilen besteht:
Sebumfilm-Reparaturkomponenten: ein Squalen, eine Fettsäure, ein Triglycerid und ein Cholesterin-Analogon;
Keratinozyten-interstitielle Komponenten: eine freie Fettsäure, ein Ceramid und ein Cholesterin-Analogon; und
synergistische Komponenten: ein beruhigender und entzündungshemmender Inhaltsstoff, ein Anti-Aging-Inhaltsstoff, ein aufhellender Inhaltsstoff und ein Hautpenetrationsmittel,
wobei in den Sebumfilm-Reparaturkomponenten das Squalen Squalan und einen Camellia-Extrakt umfasst, die Fettsäure und das Triglycerid durch eines oder mehrere von dem Camellia-Extrakt, einem Haferkernöl und einem Leindotteröl bereitgestellt werden und das Cholesterin-Analogon ein Phytosterin ist;
in den Keratinozyten-interstitiellen Komponenten die freie Fettsäure durch eines oder mehrere von dem Camellia-Extrakt, dem Haferkernöl und dem Leindotteröl bereitgestellt wird, das Ceramid durch eines oder mehrere von Ceramid 3 und dem Haferkemöl bereitgestellt wird und das Cholesterin-Analogon ein Phytosterin ist;
wobei der Camellia-Extrakt ein Camelliasamenöl, einen Camellia-Blütenextrakt und einen Camellia-Blattextrakt umfasst:
und in den synergistischen Komponenten der beruhigende und entzündungshemmende Inhaltsstoff eines oder mehrere von einer botanischen beruhigenden Kombination umfasst, umfassend eine Kombination aus mehreren Extrakten, die ausgewählt sind aus einem Matricaria recutita-Blattextrakt, einem Centella asiatica-Extrakt, einem Rosmarinus officinalis-Blattextrakt, einem Scutellaria baicalensis-Wurzelextrakt, einem Teeblattextrakt und einem Reynoutria japonica-Wurzelextrakt, Bisabolol und einem Melaleuca alternifolia-Blattöl; der Anti-Aging-Inhaltsstoff ein Tocopherol oder Vitamin E und ein Rosa rubiginosa-Samenöl umfasst; der aufhellende Inhaltsstoff ein Glycyrrhiza glabra-Wurzelextrakt ist, und das Hautpenetrationsmittel ein Melaleuca alternifolia-Blattöl ist.

2. Eine biomimetische und Camellia-extrakthaltige Hautbarrierereparaturzusammensetzung nach Anspruch 1, **dadurch gekennzeichnet, dass** die Zusammensetzung die folgenden Rohstoffe in Gewichtsprozent umfasst:
| | |
|---|---|
| Camellia-Extrakt | 10-90%, |
| Squalan | 3-60%, |
| Leindotteröl | 2-20%, |
| Rosa rubiginosa-Samenöl | 1-50%, |
| Haferkernöl | 1-20%, |
| Bisabolol | 0.1-2%, |
| Phytosterin | 0.5-5%, |
| Melaleuca alternifolia-Blattöl | 0.1-2%, |
| Tocopherol oder Vitamin E | 0.1-2%, |
| Botanische beruhigende Kombination | 0.1-5%, |
| Duftstoff oder ätherisches Öl | 0.1-2%, |
| Ceramid 3 | 0.01-1%, und |
| Glycyrrhiza glabra-Wurzelextrakt | 0.01-1%. |

3. Die biomimetische und Camellia-extrakthaltige Hautbarrierereparaturzusammensetzung nach Anspruch 2, **dadurch gekennzeichnet, dass** die botanische beruhigende Kombination die folgenden Bestandteile in Gewichtsprozent umfasst:
| | |
|---|---|
| Matricaria recutita-Blattextrakt | 5-30%, |
| Centella asiatica-Extrakt | 2-40%, |
| Rosmarinus officinalis-Blattextrakt | 3-20%, |
| Scutellaria baicalensis-Wurzelextrakt | 5-30%, |
| Teeblattextrakt | 1-20%, und |
| Reynoutria japonica-Wurzelextrakt | 10-20%. |

4. Die biomimetische und Camellia-extrakthaltige Hautbarrierereparaturzusammensetzung nach Anspruch 2, **dadurch gekennzeichnet, dass** in dem Camellia-Extrakt ein Massenverhältnis des Camelliasamenöls zu dem Camellia-Blütenextrakt zu dem Camellia-Blattextrakt 72-99.5: 0.1-15: 0.1-15 beträgt.

5. Die biomimetische und Camellia-extrakthaltige Hautbarrierereparaturzusammensetzung nach Anspruch 2, **dadurch gekennzeichnet, dass** ein Gewichtsverhältnis des Camellia-Extrakts zu dem Squalan zu dem Leindotteröl zu dem Haferkernöl zu dem Phytosterin zu dem Ceramid 3 13-16: 1: 0.5-1.5: 0.5-1.0: 0.4-0.8: 0.05-0.15 beträgt.

6. Die biomimetische und Camellia-extrakthaltige Hautbarrierereparaturzusammensetzung nach Anspruch 2, **dadurch gekennzeichnet, dass** die Zusammensetzung die folgenden Rohstoffe in Gewichtsprozent umfasst:
| | |
|---|---|
| Camellia-Extrakt | 30-75%, |
| Squalan | 4.5-35%, |
| Leindotteröl | 2.8-15%, |
| Rosa rubiginosa-Samenöl | 3.5-15%, |
| Haferkernöl | 4-10%, |
| Bisabolol | 0.1-1%, |
| Phytosterin | 0.5-3.6%, |
| Melaleuca alternifolia-Blattöl | 0.1-1.2%, |
| Tocopherol oder Vitamin E | 0.1-1.0%, |
| Botanische beruhigende Kombination | 0.5-2.0%, |
| Duftstoff oder ätherisches Öl | 0.1-1.2%, |
| Ceramid 3 | 0.01-0.8%, und |
| Glycyrrhiza glabra-Wurzelextrakt | 0.01-1%. |

7. Verwendung der biomimetischen und Camellia-extrakthaltigen Hautbarrierereparaturzusammensetzung nach Anspruch 1 oder 2 als funktioneller Rohstoff bei der Herstellung eines Kosmetikums.

8. Die Verwendung nach Anspruch 7, **dadurch gekennzeichnet, dass** das Kosmetikum ein Kosmetikum zur Reparatur und/oder Prävention von Schäden am Stratum corneum und Schäden an einem Sebumfilm der Haut ist.

## Revendications

1. Une composition de réparation de barrière cutanée biomimétique contenant un extrait de camélia, **caractérisée en ce que** la composition est constituée des parties suivantes:
des composants de réparation du film sébacé: un squalène, un acide gras, un triglycéride et un analogue de cholestérol;
des composants interstitiels des kératinocytes: un acide gras libre, un céramide et un analogue de cholestérol; et
des composants synergiques: un ingrédient apaisant et anti-inflammatoire, un ingrédient anti-âge, un ingrédient blanchissant et un agent de pénétration cutanée,
dans laquelle, dans les composants de réparation du film sébacé, le squalène comprend du squalane et un extrait de camélia, l'acide gras et le triglycéride sont fournis par un ou plusieurs éléments parmi l'extrait de camélia, une huile de noyau d'avoine et une huile de caméline, et l'analogue de cholestérol est un phytostérol;
dans les composants interstitiels des kératinocytes, l'acide gras libre est fourni par un ou plusieurs éléments parmi l'extrait de camélia, l'huile de noyau d'avoine et l'huile de caméline, le céramide est fourni par un ou plusieurs éléments parmi le céramide 3 et l'huile de noyau d'avoine, et l'analogue de cholestérol est un phytostérol;
dans laquelle l'extrait de camélia comprend une huile de graines de camélia, un extrait de fleur de camélia et un extrait de feuille de camélia:
et dans les composants synergiques, l'ingrédient apaisant et anti-inflammatoire comprend un ou plusieurs éléments parmi une combinaison botanique apaisante comprenant une combinaison de multiples extraits choisis parmi un extrait de feuille de Matricaria recutita, un extrait de Centella asiatica, un extrait de feuille de Rosmarinus officinalis, un extrait de racine de Scutellaria baicalensis, un extrait de feuille de thé et un extrait de racine de Reynoutria japonica, le bisabolol et une huile de feuille de Melaleuca alternifolia; l'ingrédient anti-âge comprend un tocophérol ou de la vitamine E, et une huile de graines de Rosa rubiginosa; l'ingrédient blanchissant est un extrait de racine de Glycyrrhiza glabra, et l'agent de pénétration cutanée est une huile de feuille de Melaleuca alternifolia.

2. Une composition de réparation de barrière cutanée biomimétique contenant un extrait de camélia, selon la revendication 1, **caractérisée en ce que** la composition comprend les matières premières suivantes en pourcentage en poids:
| | |
|---|---|
| extrait de camélia: | 10-90%, |
| squalane: | 3-60%, |
| huile de caméline: | 2-20%, |
| huile de graines de Rosa rubiginosa: | 1-50%, |
| huile de noyau d'avoine: | 1-20%, |
| bisabolol: | 0.1-2%, |
| phytostérol: | 0.5-5%, |
| huile de feuille de Melaleuca alternifolia: | 0.1-2%, |
| tocophérol ou vitamine E: | 0.1-2%, |
| combinaison botanique apaisante: | 0.1-5%, |
| parfum ou huile essentielle: | 0.1-2%, |
| céramide 3: | 0.01-1%, et |
| extrait de racine de Glycyrrhiza glabra: | 0.01-1%. |

3. La composition de réparation de barrière cutanée biomimétique contenant un extrait de camélia selon la revendication 2, **caractérisée en ce que** la combinaison botanique apaisante comprend les parties suivantes en pourcentage en poids:
| | |
|---|---|
| extrait de feuille de Matricaria recutita: | 5-30%, |
| extrait de Centella asiatica: | 2-40%, |
| extrait de feuille de Rosmarinus officinalis: | 3-20%, |
| extrait de racine de Scutellaria baicalensis: | 5-30%, |
| extrait de feuille de thé: | 1-20%, et |
| extrait de racine de Reynoutria japonica: | 10-20%. |

4. La composition de réparation de barrière cutanée biomimétique contenant un extrait de camélia selon la revendication 2, **caractérisée en ce que**, dans l'extrait de camélia, un rapport massique de l'huile de graines de camélia à l'extrait de fleur de camélia et à l'extrait de feuille de camélia est de 72-99.5: 0.1-15: 0.1-15.

5. La composition de réparation de barrière cutanée biomimétique contenant un extrait de camélia selon la revendication 2, **caractérisée en ce qu'**un rapport pondéral de l'extrait de camélia au squalane, à l'huile de caméline, à l'huile de noyau d'avoine, au phytostérol et au céramide 3 est de 13-16: 1: 0.5-1.5: 0.5-1.0: 0.4-0.8: 0.05-0.15.

6. La composition de réparation de barrière cutanée biomimétique contenant un extrait de camélia selon la revendication 2, **caractérisée en ce que** la composition comprend les matières premières suivantes en pourcentage en poids:
| | |
|---|---|
| extrait de camélia: | 30-75%, |
| squalane: | 4.5-35%, |
| huile de caméline: | 2.8-15%, |
| huile de graines de Rosa rubiginosa: | 3.5-15%, |
| huile de noyau d'avoine: | 4-10%, |
| bisabolol: | 0.1-1%, |
| phytostérol: | 0.5-3.6%, |
| huile de feuille de Melaleuca alternifolia: | 0.1-1.2%, |
| tocophérol ou vitamine E: | 0.1-1.0%, |
| combinaison botanique apaisante: | 0.5-2.0%, |
| parfum ou huile essentielle: | 0.1-1.2%, |
| céramide 3: | 0.01-0.8%, et |
| extrait de racine de Glycyrrhiza glabra: | 0.01-1%. |

7. Utilisation de la composition de réparation de barrière cutanée biomimétique contenant un extrait de camélia selon la revendication 1 ou 2 comme matière première fonctionnelle dans la préparation d'un cosmétique.

8. L'utilisation selon la revendication 7, **caractérisée en ce que** le cosmétique est un cosmétique destiné à réparer et/ou à prévenir les dommages à la couche cornée et les dommages à un film sébacé de la peau.
